# EUROPEAN PATENT SPECIFICATION

(11) **EP 4 033 988 B1**
(45) Date of publication and mention of the grant of the patent: **06.08.2025**
(21) Application number: 20790105.9
(22) Date of filing: 24.09.2020
(51) Int. Cl.: G06N 3/08, G06T 7/12, A61B 8/08, A61B 8/12, A61B 8/00

(54) **INTRAVASCULAR ULTRASOUND IMAGING AND CALCIUM DETECTION METHODS**
INTRAVASKULÄRE ULTRASCHALLBILDGEBUNG UND VERFAHREN ZUR CALCIUMDETEKTION
IMAGERIE ULTRASONORE INTRAVASCULAIRE ET PROCÉDÉS DE DÉTECTION DE CALCIUM

(30) Priority: 26.09.2019 US 201962906546 P
(43) Date of publication of application: 03.08.2022
(73) Proprietor: Boston Scientific Scimed, Inc., Maple Grove, MN 55311 (US)
(72) Inventor: CAI, Anming He, San Jose, California 95120 (US); LI, Wenguang, Los Gatos, California 95032 (US)
(74) Representative: Vossius & Partner Patentanwälte Rechtsanwälte mbB
(86) International application number: PCT/US2020/052517
(87) International publication number: WO 2021/062006

(56) References cited:
- CN-A- 107 909 585
- CN-A- 110 070 529
- KR-A- 20170 113 251
- US-A1- 2015 073 279

## Description

### Technical Field

The present disclosure pertains to intravascular ultrasound imaging.

### Background

A wide variety of medical devices have been developed for medical use, for example, intravascular use. Some of these devices include intravascular ultrasound imaging devices. In addition, methods for intravascular ultrasound imaging have been developed. Of these devices and methods, each has certain advantages and disadvantages. There is an ongoing need to provide alternative devices as well as alternative methods.

Methods for processing intra-vascular images are known from documents CN 110 070 529 A, US 2015/073279 A1, CN 107 909 585 A, and KR 2017 0113251 A.

### Brief Summary

The invention is set out in the independent claims. Particular embodiments of the invention are set out in the dependent claims. Any subject matter contained herein that does not fall within the scope of the appended claims is considered as being useful for understanding the invention.

This disclosure provides design and use alternatives for medical devices as well as methods, for example methods that include intravascular ultrasound imaging.

A method for processing intravascular ultrasound images is disclosed. The method comprises: collecting one or more ultrasound images of a blood vessel; segmenting the ultrasound images with a processor; wherein the processor includes a deep neural network; and wherein segmenting includes identifying one or more of a lumen border of the blood vessel and a media border for media within the blood vessel.

Alternatively or additionally to any of the embodiments above, segmenting includes identifying the lumen border.

Segmenting includes identifying the media border.

Alternatively or additionally to any of the embodiments above, the deep neural network is trained to identify the lumen border.

Alternatively or additionally to any of the embodiments above, the deep neural network is trained to identify the media border.

Alternatively or additionally to any of the embodiments above, the deep neural network is trained to identify the lumen border and is trained to identify the media border.

Further comprising displaying a first image of the one or more ultrasound images on a display device.

Alternatively or additionally to any of the embodiments above, further comprising displaying a numerical indication of stenosis area for the first image on the display device.

Alternatively or additionally to any of the embodiments above, the numerical indication of stenosis area is displayed on or adjacent to the first image.

Alternatively or additionally to any of the embodiments above, further comprising displaying a numerical indication of plaque burden for the first image on the display device.

Alternatively or additionally to any of the embodiments above, the numerical indication of plaque burden is displayed on or adjacent to the first image.

Further comprising displaying a visual representation of a calcification angle on the display device.

Alternatively or additionally to any of the embodiments above, the visual representation of the calcification angle includes an arc disposed along a boundary region of the first image.

Alternatively or additionally to any of the embodiments above, further comprising displaying a visual representation of a calcification arc on the display device.

Alternatively or additionally to any of the embodiments above, the visual representation of the calcification arc includes an arc disposed along a boundary region of the first image.

A method for processing intravascular ultrasound images is disclosed. The method comprises: collecting a plurality of ultrasound images of a blood vessel; segmenting the ultrasound images with a processor; wherein the processor includes a deep neural network; and wherein segmenting includes identifying a lumen border of the blood vessel, a cross-sectional area of the blood vessel, a media border for media within the blood vessel, a cross-sectional area of the media, a calcification angle of a calcified lesion within the blood vessel, a side branch location, or combinations thereof.

Alternatively or additionally to any of the embodiments above, collecting a plurality of ultrasound images of a blood vessel includes collecting the plurality of ultrasound images with an intravascular ultrasound catheter system.

Alternatively or additionally to any of the embodiments above, collecting a plurality of ultrasound images of a blood vessel includes collecting cross-sectional images of the blood vessel.

Alternatively or additionally to any of the embodiments above, segmenting the ultrasound images with a processor includes segmenting with artificial intelligence.

Alternatively or additionally to any of the embodiments above, segmenting the ultrasound images with a processor includes segmenting with the deep neural network.

Alternatively or additionally to any of the embodiments above, segmenting the ultrasound images with a processor includes quantitative analysis.

Alternatively or additionally to any of the embodiments above, segmenting the ultrasound images with a processor includes image classification.

Alternatively or additionally to any of the embodiments above, segmenting the ultrasound images with a processor includes identification of a lesion within the blood vessel.

Alternatively or additionally to any of the embodiments above, segmenting the ultrasound images with a processor includes stent detection.

Alternatively or additionally to any of the embodiments above, segmenting includes identifying a lumen border of the blood vessel.

Alternatively or additionally to any of the embodiments above, segmenting includes identifying a cross-sectional area of the blood vessel.

Alternatively or additionally to any of the embodiments above, segmenting includes identifying a media border for media within the blood vessel.

Alternatively or additionally to any of the embodiments above, segmenting includes identifying a cross-sectional area of the media.

Alternatively or additionally to any of the embodiments above, segmenting includes identifying a calcification angle of a calcified lesion within the blood vessel.

Alternatively or additionally to any of the embodiments above, segmenting includes identifying a side branch location.

Alternatively or additionally to any of the embodiments above, further comprising displaying a first cross-sectional image of the blood vessel on a display.

Alternatively or additionally to any of the embodiments above, further comprising displaying a second cross-sectional image of the blood vessel on the display.

Alternatively or additionally to any of the embodiments above, further comprising displaying a numerical indication of stenosis area for the first cross-sectional image, the second cross-sectional image, or both on the display.

Alternatively or additionally to any of the embodiments above, further comprising displaying a numerical indication of plaque burden for the first cross-sectional image, the second cross-sectional image, or both on the display.

Alternatively or additionally to any of the embodiments above, further comprising displaying a visual representation of a calcification angle.

Alternatively or additionally to any of the embodiments above, the visual representation of the calcification angle includes an arc disposed along a boundary region of the first cross-sectional image.

Alternatively or additionally to any of the embodiments above, further comprising displaying a visual representation of a calcification arc.

Alternatively or additionally to any of the embodiments above, the visual representation of the calcification angle includes an arc disposed along a boundary region of the first cross-sectional image.

A method for displaying images of a blood vessel is disclosed. The method comprises: receiving with a processing unit electrical signals from a transducer coupled to a catheter as the transducer rotates and moves longitudinally along a lumen of a blood vessel; processing the received electrical signals to form a series of cross-sectional images; wherein the cross-sectional images are longitudinally-offset from one another along the lumen; displaying one or more of the cross-sectional images on a display; and displaying a lumen diameter profile, a vessel diameter profile, or both on the display.

Alternatively or additionally to any of the embodiments above, the lumen diameter profile is configured to allow a clinician to identify a minimal lumen area within the blood vessel.

Alternatively or additionally to any of the embodiments above, the vessel diameter profile is configured to allow a clinician to identify side branches extending from the blood vessel.

Alternatively or additionally to any of the embodiments above, further comprising displaying a two-dimensional representation of calcium coverage within the blood vessel on the display.

Alternatively or additionally to any of the embodiments above, further comprising displaying a two-dimensional calcium map on the display.

Alternatively or additionally to any of the embodiments above, further comprising displaying a three-dimensional representation of calcium coverage within the blood vessel on the display.

Alternatively or additionally to any of the embodiments above, further comprising displaying a three-dimensional calcium map on the display.

Alternatively or additionally to any of the embodiments above, further comprising displaying a visual representation of a calcification angle on the display.

Alternatively or additionally to any of the embodiments above, the visual representation of the calcification angle includes an arc disposed along a boundary region of one of the cross-sectional images.

Alternatively or additionally to any of the embodiments above, further comprising displaying a visual representation of a calcification arc on the display.

Alternatively or additionally to any of the embodiments above, the visual representation of the calcification arc includes an arc disposed along a boundary region of one of the cross-sectional images.

A method for graphically displaying calcification of a blood vessel is disclosed. The method comprises: collecting a plurality of ultrasound images of a blood vessel; segmenting the images to generate a calcification angle for each of the plurality of ultrasound images; and displaying a visual representation of the calcification angles.

Alternatively or additionally to any of the embodiments above, the visual representation of the calcification angles includes an arc disposed along a boundary region of one of the images.

Alternatively or additionally to any of the embodiments above, the visual representation of the calcification angles includes a two-dimensional calcium map.

Alternatively or additionally to any of the embodiments above, the visual representation of the calcification angles includes a three-dimensional calcium map.

A method for determining a treatment strategy for treating an intravascular lesion in a blood vessel is disclosed. The method comprises: determining a first numerical calcium score by observing a visual representation of a calcium arc of the intravascular lesion, a two-dimensional calcium map of the blood vessel, a three-dimensional calcium map of the blood vessel, or combinations thereof; determining a second numerical calcium score by observing a fluoroscopic image of the blood vessel; determining a third numerical calcium score by measuring a length of the intravascular lesion; calculating a sum of the first numerical calcium score, the second numerical calcium score, and a third calcium score; and selecting a treatment modality based on the sum.

A method for using artificial intelligence to process intravascular ultrasound images is disclosed.

A method for using artificial intelligence to process intravascular ultrasound images as disclosed and/or claimed herein is disclosed.

A method for determining an arc size for an amount of calcification in a blood vessel is disclosed.

Alternatively or additionally to any of the embodiments above, further comprising displaying the amount of calcification on a display device.

Alternatively or additionally to any of the embodiments above, displaying the amount of calcification on a display device includes displaying an arc along a boundary region of a cross-sectional ultrasound image of the blood vessel.

Alternatively or additionally to any of the embodiments above, displaying the amount of calcification on a display device includes a two-dimensional calcium map.

Alternatively or additionally to any of the embodiments above, displaying the amount of calcification on a display device includes a three-dimensional calcium map.

A method for processing intravascular ultrasound images is disclosed. The method comprises: collecting one or more ultrasound images of a blood vessel; segmenting the ultrasound images with a processor; and wherein segmenting includes identifying one or more of a lumen border of the blood vessel and a media border for media within the blood vessel.

Alternatively or additionally to any of the embodiments above, segmenting includes identifying the lumen border.

Alternatively or additionally to any of the embodiments above, segmenting includes identifying the media border.

Alternatively or additionally to any of the embodiments above, the processor includes a deep neural network.

Alternatively or additionally to any of the embodiments above, the deep neural network is trained to identify the lumen border.

Alternatively or additionally to any of the embodiments above, the deep neural network is trained to identify the media border.

Alternatively or additionally to any of the embodiments above, the deep neural network is trained to identify the lumen border and is trained to identify the media border.

A method for processing intravascular ultrasound images is disclosed. The method comprises: collecting a plurality of ultrasound images of a blood vessel; segmenting the ultrasound images with a processor; and wherein segmenting includes identifying a lumen border of the blood vessel, a cross-sectional area of the blood vessel, a media border for media within the blood vessel, a cross-sectional area of the media, a calcification angle of a calcified lesion within the blood vessel, a side branch location, or combinations thereof.

Alternatively or additionally to any of the embodiments above, the processor includes a deep neural network.

Alternatively or additionally to any of the embodiments above, the deep neural network is trained to identify the lumen border.

Alternatively or additionally to any of the embodiments above, the deep neural network is trained to identify the media border.

Alternatively or additionally to any of the embodiments above, the deep neural network is trained to identify the lumen border and is trained to identify the media border.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 schematically depicts an example intravascular ultrasound system.
FIG. 2 is a perspective view of an example intravascular ultrasound catheter system.
FIG. 3 is a side view of a portion of an example intravascular ultrasound catheter system.
FIG. 4 is a flow chart illustrating an example method for processing images.
FIG. 5 is a flow chart illustrating an example method for processing images.
FIG. 6 schematically illustrates a process for image segmentation.
FIG. 7 illustrates an example of display output.
FIG. 8 illustrates an example of display output.
FIG. 9 illustrates an example of display output.
FIGS. 10-12 illustrate some example of display output(s).

While the disclosure is amenable to various modifications and alternative forms, specifics thereof have been shown by way of example in the drawings and will be described in detail. It should be understood, however, that the intention is not to limit the invention to the particular embodiments described.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Ultrasound devices insertable into patients have proven diagnostic capabilities for a variety of diseases and disorders. For example, intravascular ultrasound ("IVUS") imaging systems may be used as an imaging modality for diagnosing blocked blood vessels and providing information to aid medical practitioners in selecting and placing stents and other devices to restore or increase blood flow. IVUS imaging systems may also be used to diagnose atheromatous plaque build-up at particular locations within blood vessels. IVUS imaging systems may also be used to determine the existence of an intravascular obstruction or stenosis, as well as the nature and degree of the obstruction or stenosis. IVUS imaging systems may also be used to visualize segments of a vascular system that may be difficult to visualize using other intravascular imaging techniques, such as angiography, due to, for example, movement (e.g., a beating heart) or obstruction by one or more structures (e.g., one or more blood vessels not desired to be imaged). IVUS imaging systems may also be used to monitor or assess ongoing intravascular treatments, such as angiography and stent placement in real (or almost real) time. Moreover, IVUS imaging systems may be used to monitor one or more heart chambers.

IVUS imaging systems have been developed to provide a diagnostic tool for visualizing a variety is diseases or disorders. An IVUS imaging system may include a control module (with a pulse generator, an image processor, and a monitor), a catheter, and one or more transducers disposed in the catheter. The transducer-containing catheter may be positioned in a lumen or cavity within, or in proximity to, a region to be imaged, such as a blood vessel wall or patient tissue in proximity to a blood vessel wall. The pulse generator in the control module may generate electrical pulses that are delivered to the one or more transducers and transformed to acoustic pulses that are transmitted through patient tissue. Reflected pulses of the transmitted acoustic pulses may be absorbed by the one or more transducers and transformed to electric pulses. The transformed electric pulses may be delivered to the image processor and converted to an image displayable on the monitor.

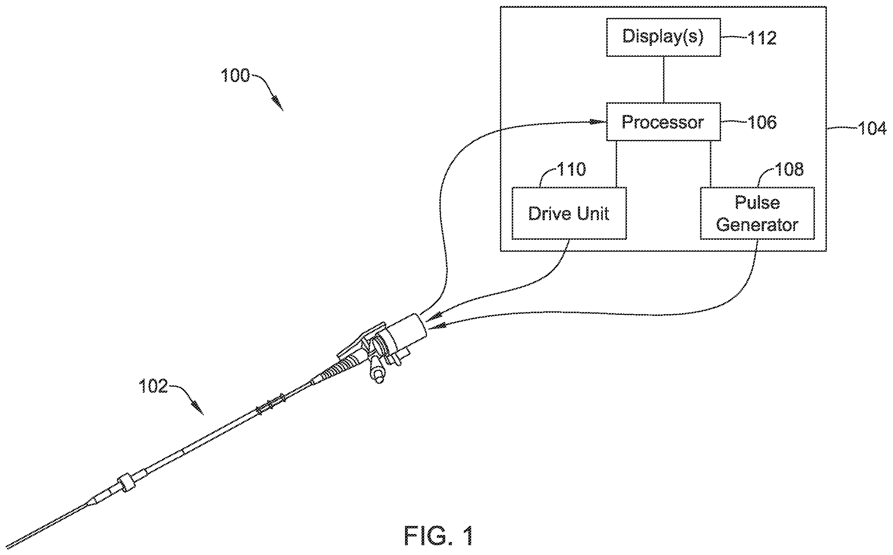

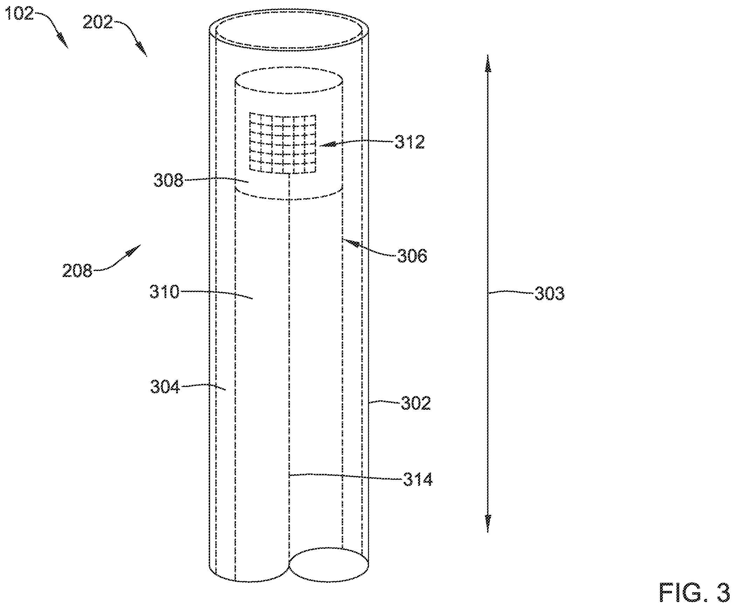

FIG. 1 illustrates schematically an example IVUS imaging system 100. The IVUS imaging system 100 includes a catheter 102 that is coupleable to a processing unit or control module 104. The control module 104 may include, for example, a processor 106, a pulse generator 108, a drive unit 110, and one or more displays 112. In some instances, the pulse generator 108 forms electric pulses that may be input to one or more transducers (312 in FIG. 3) disposed in the catheter 102.

In some instances, mechanical energy from the drive unit 110 may be used to drive an imaging core (306 in FIG. 3) disposed in the catheter 102. In some instances, electric signals transmitted from the one or more transducers (312 in FIG. 3) may be input to the processor 106 for processing. In some instances, the processed electric signals from the one or more transducers (312 in FIG. 3) can be displayed as one or more images on the one or more displays 112. For example, a scan converter can be used to map scan line samples (e.g., radial scan line samples, or the like) to a two-dimensional Cartesian grid to display the one or more images on the one or more displays 112.

In some instances, the processor 106 may also be used to control the functioning of one or more of the other components of the control module 104. For example, the processor 106 may be used to control at least one of the frequency or duration of the electrical pulses transmitted from the pulse generator 108, the rotation rate of the imaging core (306 in FIG. 3) by the drive unit 110, the velocity or length of the pullback of the imaging core (306 in FIG. 3) by the drive unit 110, or one or more properties of one or more images formed on the one or more displays 112.

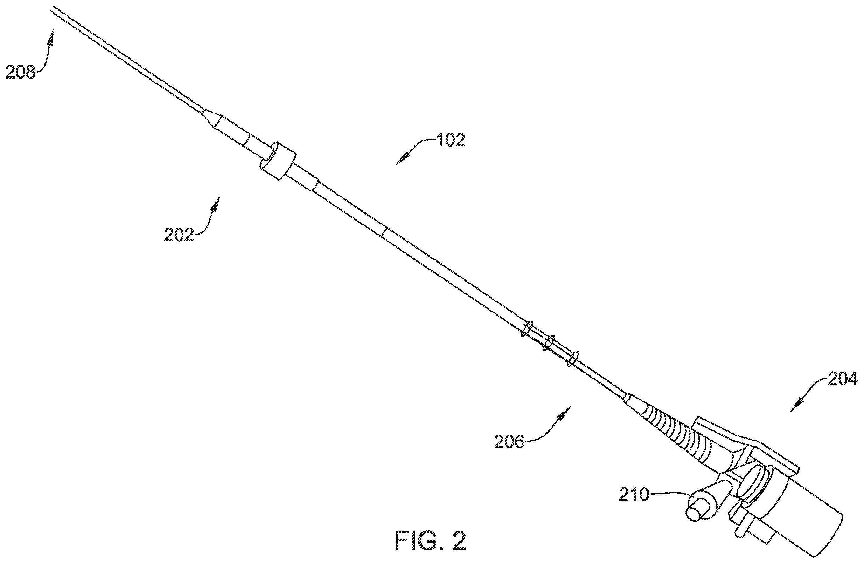

FIG. 2 is a schematic side view of one embodiment of the catheter 102 of the IVUS imaging system (100 in FIG. 1). The catheter 102 includes an elongated member 202 and a hub 204. The elongated member 202 includes a proximal end 206 and a distal end 208. In FIG. 2, the proximal end 206 of the elongated member 202 is coupled to the catheter hub 204 and the distal end 208 of the elongated member is configured and arranged for percutaneous insertion into a patient. Optionally, the catheter 102 may define at least one flush port, such as flush port 210. The flush port 210 may be defined in the hub 204. The hub 204 may be configured and arranged to couple to the control module (104 in FIG 1). In some instances, the elongated member 202 and the hub 204 are formed as a unitary body. In other instances, the elongated member 202 and the catheter hub 204 are formed separately and subsequently assembled together.

FIG. 3 is a schematic perspective view of one embodiment of the distal end 208 of the elongated member 202 of the catheter 102. The elongated member 202 includes a sheath 302 with a longitudinal axis 303 and a lumen 304. An imaging core 306 is disposed in the lumen 304. The imaging core 306 includes an imaging device 308 coupled to a distal end of a driveshaft 310 that is rotatable either manually or using a computer-controlled drive mechanism. One or more transducers 312 may be mounted to the imaging device 308 and employed to transmit and receive acoustic signals. The sheath 302 may be formed from any flexible, biocompatible material suitable for insertion into a patient. Examples of suitable materials include, for example, polyethylene, polyurethane, plastic, spiral-cut stainless steel, nitinol hypotube, and the like or combinations thereof.

In some instances, for example as shown in Figure 3, an array of transducers 312 are mounted to the imaging device 308. Alternatively, a single transducer may be employed. Any suitable number of transducers 312 can be used. For example, there can be two, three, four, five, six, seven, eight, nine, ten, twelve, fifteen, sixteen, twenty, twenty-five, fifty, one hundred, five hundred, one thousand, or more transducers. As will be recognized, other numbers of transducers may also be used. When a plurality of transducers 312 are employed, the transducers 312 can be configured into any suitable arrangement including, for example, an annular arrangement, a rectangular arrangement, or the like.

The one or more transducers 312 may be formed from materials capable of transforming applied electrical pulses to pressure distortions on the surface of the one or more transducers 312, and vice versa. Examples of suitable materials include piezoelectric ceramic materials, piezocomposite materials, piezoelectric plastics, barium titanates, lead zirconate titanates, lead metaniobates, polyvinylidene fluorides, and the like. Other transducer technologies include composite materials, single-crystal composites, and semiconductor devices (e.g., capacitive micromachined ultrasound transducers ("cMUT"), piezoelectric micromachined ultrasound transducers ("pMUT"), or the like).

The pressure distortions on the surface of the one or more transducers 312 form acoustic pulses of a frequency based on the resonant frequencies of the one or more transducers 312. The resonant frequencies of the one or more transducers 312 may be affected by the size, shape, and material used to form the one or more transducers 312. The one or more transducers 312 may be formed in any shape suitable for positioning within the catheter 102 and for propagating acoustic pulses of a desired frequency in one or more selected directions. For example, transducers may be disc-shaped, block-shaped, rectangular-shaped, oval-shaped, and the like. The one or more transducers may be formed in the desired shape by any process including, for example, dicing, dice and fill, machining, microfabrication, and the like.

As an example, each of the one or more transducers 312 may include a layer of piezoelectric material sandwiched between a matching layer and a conductive backing material formed from an acoustically absorbent material (e.g., an epoxy substrate with tungsten particles). During operation, the piezoelectric layer may be electrically excited to cause the emission of acoustic pulses.

The one or more transducers 312 can be used to form a radial cross-sectional image of a surrounding space. Thus, for example, when the one or more transducers 312 are disposed in the catheter 102 and inserted into a blood vessel of a patient, the one more transducers 312 may be used to form an image of the walls of the blood vessel and tissue surrounding the blood vessel.

The imaging core 306 is rotated about the longitudinal axis 303 of the catheter 102. As the imaging core 306 rotates, the one or more transducers 312 emit acoustic signals in different radial directions (e.g., along different radial scan lines). For example, the one or more transducers 312 can emit acoustic signals at regular (or irregular) increments, such as 256 radial scan lines per revolution, or the like. It will be understood that other numbers of radial scan lines can be emitted per revolution, instead.

When an emitted acoustic pulse with sufficient energy encounters one or more medium boundaries, such as one or more tissue boundaries, a portion of the emitted acoustic pulse is reflected back to the emitting transducer as an echo pulse. Each echo pulse that reaches a transducer with sufficient energy to be detected is transformed to an electrical signal in the receiving transducer. The one or more transformed electrical signals are transmitted to the control module (104 in FIG. 1) where the processor 106 processes the electrical-signal characteristics to form a displayable image of the imaged region based, at least in part, on a collection of information from each of the acoustic pulses transmitted and the echo pulses received. In some instances, the rotation of the imaging core 306 is driven by the drive unit 110 disposed in the control module (104 in FIG. 1). In alternate embodiments, the one or more transducers 312 are fixed in place and do not rotate. In which case, the driveshaft 310 may, instead, rotate a mirror that reflects acoustic signals to and from the fixed one or more transducers 312.

When the one or more transducers 312 are rotated about the longitudinal axis 303 of the catheter 102 emitting acoustic pulses, a plurality of images can be formed that collectively form a radial cross-sectional image (e.g., a tomographic image) of a portion of the region surrounding the one or more transducers 312, such as the walls of a blood vessel of interest and tissue surrounding the blood vessel. The radial cross-sectional image can, optionally, be displayed on one or more displays 112. The at least one of the imaging core 306 can be either manually rotated or rotated using a computer-controlled mechanism.

The imaging core 306 may also move longitudinally along the blood vessel within which the catheter 102 is inserted so that a plurality of cross-sectional images may be formed along a longitudinal length of the blood vessel. During an imaging procedure the one or more transducers 312 may be retracted (e.g., pulled back) along the longitudinal length of the catheter 102. The catheter 102 can include at least one telescoping section that can be retracted during pullback of the one or more transducers 312. In some instances, the drive unit 110 drives the pullback of the imaging core 306 within the catheter 102. The drive unit 110 pullback distance of the imaging core can be any suitable distance including, for example, at least 5 cm, 10 cm, 15 cm, 20 cm, 25 cm, or more. The entire catheter 102 can be retracted during an imaging procedure either with or without the imaging core 306 moving longitudinally independently of the catheter 102.

A stepper motor may, optionally, be used to pull back the imaging core 306. The stepper motor can pull back the imaging core 306 a short distance and stop long enough for the one or more transducers 306 to capture an image or series of images before pulling back the imaging core 306 another short distance and again capturing another image or series of images, and so on.

The quality of an image produced at different depths from the one or more transducers 312 may be affected by one or more factors including, for example, bandwidth, transducer focus, beam pattern, as well as the frequency of the acoustic pulse. The frequency of the acoustic pulse output from the one or more transducers 312 may also affect the penetration depth of the acoustic pulse output from the one or more transducers 312. In general, as the frequency of an acoustic pulse is lowered, the depth of the penetration of the acoustic pulse within patient tissue increases. In some instances, the IVUS imaging system 100 operates within a frequency range of 5 MHz to 100 MHz.

One or more conductors 314 can electrically couple the transducers 312 to the control module 104 (see, for example, FIG. 1). In which case, the one or more conductors 314 may extend along a longitudinal length of the rotatable driveshaft 310.

The catheter 102 with one or more transducers 312 mounted to the distal end 208 of the imaging core 308 may be inserted percutaneously into a patient via an accessible blood vessel, such as the femoral artery, femoral vein, or jugular vein, at a site remote from the selected portion of the selected region, such as a blood vessel, to be imaged. The catheter 102 may then be advanced through the blood vessels of the patient to the selected imaging site, such as a portion of a selected blood vessel.

An image or image frame ("frame") can be generated each time one or more acoustic signals are output to surrounding tissue and one or more corresponding echo signals are received by the imager 308 and transmitted to the processor 106. Alternatively, an image or image frame can be a composite of scan lines from a full or partial rotation of the imaging core or device. A plurality (e.g., a sequence) of frames may be acquired over time during any type of movement of the imaging device 308. For example, the frames can be acquired during rotation and pullback of the imaging device 308 along the target imaging location. It will be understood that frames may be acquired both with or without rotation and with or without pullback of the imaging device 308. Moreover, it will be understood that frames may be acquired using other types of movement procedures in addition to, or in lieu of, at least one of rotation or pullback of the imaging device 308.

In some instances, when pullback is performed, the pullback may be at a constant rate, thus providing a tool for potential applications able to compute longitudinal vessel/plaque measurements. In some instances, the imaging device 308 is pulled back at a constant rate of at least 0.3 mm/s. In some instances, the imaging device 308 is pulled back at a constant rate of at least 0.4 mm/s. In some instances, the imaging device 308 is pulled back at a constant rate of at least 0.5 mm/s. In some instances, the imaging device 308 is pulled back at a constant rate of at least 0.6 mm/s. In some instances, the imaging device 308 is pulled back at a constant rate of at least 0.7 mm/s. In some instances the imaging device 308 is pulled back at a constant rate of at least 0.8 mm/s.

In some instances, the one or more acoustic signals are output to surrounding tissue at constant intervals of time. In some instances, the one or more corresponding echo signals are received by the imager 308 and transmitted to the processor 106 at constant intervals of time. In some instances, the resulting frames are generated at constant intervals of time.

At least some conventional IVUS imaging systems display only a single (e.g., cross-sectional, longitudinal, or the like) image during, or after, an IVUS procedure, such as a pull-back procedure. It may, however, be useful to concurrently display, in real-time during the IVUS procedure (e.g., a pull-back procedure), at least two images, such as the most recently processed image and a previously-obtained image that has some particular or selected image characteristic (e.g., maximum or minimum lumen area or diameter).

Some diagnostic and/or therapeutic interventions may include the analysis of images generated by the IVUS imaging system. This analysis, however, may require a substantial amount of training/experience in order to efficiently interpret the images. Further, due to the frequent presence of speckles on IVUS images, automated analysis and/or evaluation may also be challenging. Disclosed herein are methods for processing and/or analyzing images such as images generated with/by an IVUS imaging system. Such methods may utilize machine learning, artificial intelligence, deep neural networks, and/or the like to improve the processing and/or analysis of images generated with/by an IVUS imaging system.

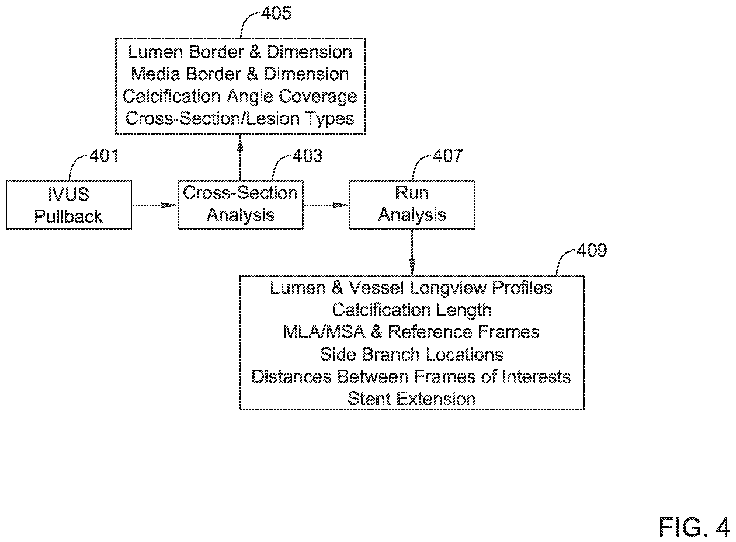

FIG. 4 is a flow chart illustrating an overview or framework of an example process. The process may include the generation and/or collection of images of a blood vessel (e.g., IVUS images, cross-sectional images, etc. generated via an IVUS pullback procedure) at box 401. The generated/collected images may be subjected to a cross-sectional analysis at box 403. The cross-sectional analysis may include processing and/or segmentation of the images using deep learning networks (e.g., deep neural networks such as the U-Net deep neural network) to obtain image segmentation for quantitative analysis and image classification for automated identification of lesion type, stent detection, and the like. For example, output from cross-sectional analysis, marked at box 405, may include identification of the lumen border, identification of the lumen dimensions, identification of the media border (e.g., identification of a media border for media within the blood vessel), identification of the media dimensions, identification of the calcification angle/arc, identification of the calcification coverage, identification of the lesion types, and/or the like. In addition to identifying such border/dimension, the output may be displayed on a display unit in a suitable format (e.g., graphically, numerically, as a real or schematic image, with words or symbols, etc.). In some instances, multiple images of an IVUS pullback or "run" may be analyzed at box 407. The output of this run analysis, marked at box 409, may include a lumen profile (e.g., including, for example, a longitudinal cross-section or "long view"), a vessel profile (e.g., including, for example, a longitudinal cross-section or "long view"), a representation (e.g., visualization or image, numerical visualization, graphical visualization, and/or the like) of the calcification length, the depiction/display of reference frames (e.g., such as the minimal lumen area or "MLA", the minimal stent area or "MSA", or the like), a representation (e.g., visualization or image, numerical visualization, graphical visualization, and/or the like) of side branch location, a representation (e.g., visualization or image, numerical visualization, graphical visualization, and/or the like) of the distance between two frames of interest, a representation (e.g., visualization or image, numerical visualization, graphical visualization, and/or the like) of stent extension, combinations thereof, and/or the like. This may also include analyzing the images with a deep neural network (e.g., such as UNet deep neural network) and/or machine learning and/or artificial intelligence.

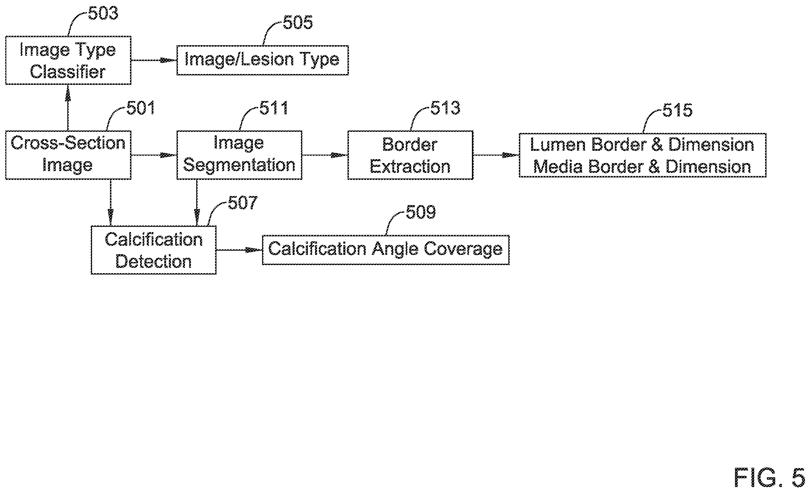

FIG. 5 is a flow chart depicting an example process by which an image (e.g., IVUS images, cross-sectional images, etc. generated via an IVUS pullback procedure) may be processed/segmented. For example, an example cross-sectional image (e.g., at box 501) or a group/collection of images may undergo image classification (e.g., at box 503), for example to identify the type of lesion (e.g., at box 505). In some instances, the output may be displayed on a display unit in a suitable format (e.g., graphically, numerically, as a real or schematic image, with words or symbols, etc.). In some instances, an image (e.g., at box 501) may undergo calcification detection (e.g., at box 507) to identify the calcium/calcification angle or arc coverage (e.g., at box 509).

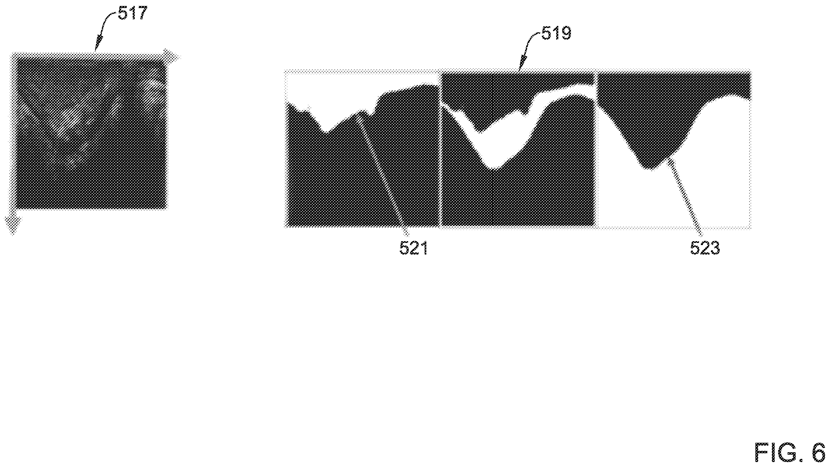

In some instances, an image (e.g., at box 501) may undergo image segmentation (e.g., at box 511). This may include the extraction of borders (e.g., at box 513) in order to identify the lumen border, identify the dimensions of the lumen, identify the media border, identify the dimensions of the media, and/or the like (e.g., at box 515). Some example output (e.g., which may be displayed on a display unit) of image segmentation is shown in FIG. 6. An image of the blood vessel 517 may undergo image segmentation. This may include analyzing the image with a deep neural network (e.g., such as the U-Net deep neural network and/or other networks trained to identify a lumen border, a media border, or both) and/or machine learning and/or artificial intelligence. This may result in a visualization 519 where the lumen border 521 and the media border 523 are identified.

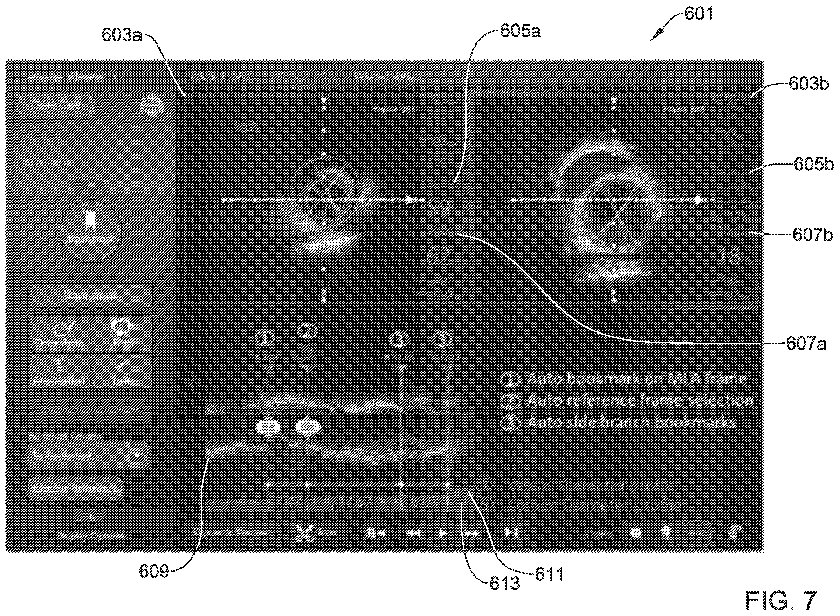

FIG. 7 is an example display output 601 that may be shown on a display unit. A number of features may be shown. For one or more representative images 603a, 603b of cross-sections of a blood vessel may be shown. In some instances, a numerical indication of stenosis area 605a, 605b and/or a numerical indication of plaque burden 607a, 607b may be shown next to or adjacent to the images 603a, 603b. In some instances, a representation/visualization of the longitudinal cross-section of the blood vessel 609 may be shown. In some instances, a visual representation of the calcification angle/arc may be shown on the display output 601. For example, the visual representation of the calcification angle/arc may include an arc or arcing line disposed along a boundary region of the images 603a, 603b. In some instances, the display may include a representation/visualization of the vessel diameter profile 611 and/or the lumen diameter profile 613. These profiles 611, 613 may make it more efficient for a clinician to identify regions of the vessel with the smallest area (e.g., the MLA) and/or other areas of interest within the vessel.

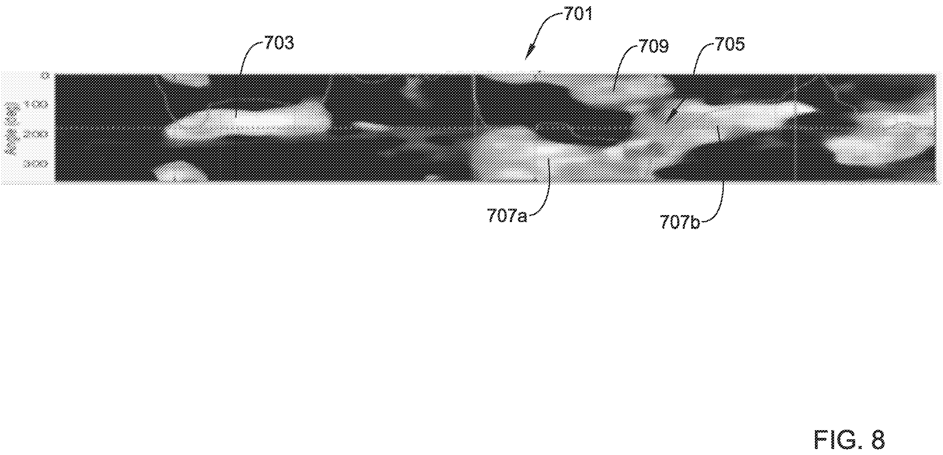

FIG. 8 is another example display output 701 that may be shown on a display unit. This display output 701 may take the form of a calcium map (e.g., a two-dimensional calcium map that depicts calcification angle/arc). In this example, the longitudinal position is shown along the X-axis and calcification angle/arc is shown along the Y-axis. Several bright spots, indicating the presence of calcification, are shown on the calcium map. For example, a first calcification spot 703 and a second calcification spot 705 are shown. The first calcification spot 703 may have a relatively long longitudinal component but with a smaller/shorter calcification angle/arc whereas the second calcification spot 705 may include longitudinal sections 707a, 707b (e.g., having a relatively long longitudinal component) and a circumferential section 709 (e.g., having a larger, more circumferential calcification angle/arc).

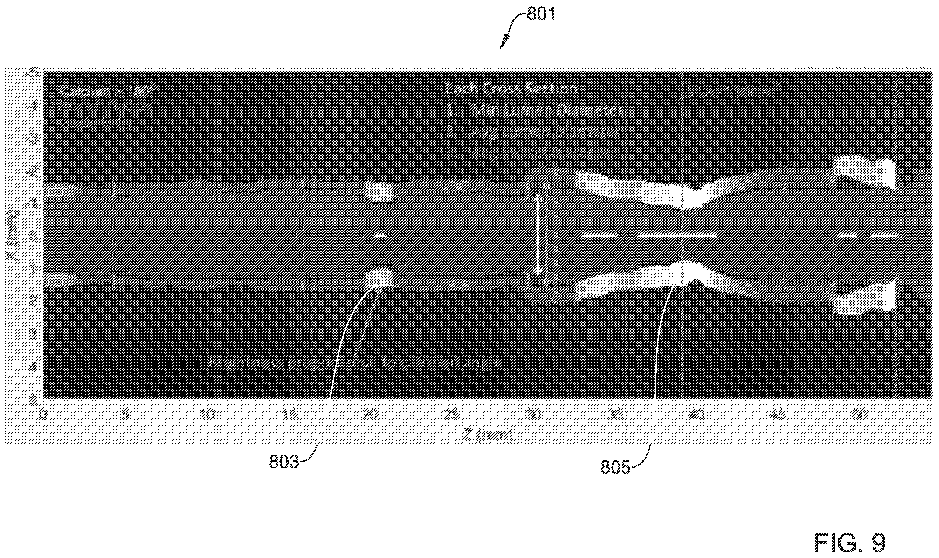

FIG. 9 is another example display output 801 that may be shown on a display unit. This display output 801 may take the form of a calcium map (e.g., a two-dimensional calcium map that depicts calcification angle/arc). In this example, the longitudinal position is shown along the X-axis and calcification angle/arc is shown using variable brightness and/or differences in color/grayscale. In this example, a number of bright spots, indicating the presence of calcification, are shown on the calcium map. For example, a first calcification spot 803 and a second calcification spot 805 are shown. The first calcification spot 803 may have a first brightness whereas the second calcification spot 805 may have a second brightness that is brighter than the first brightness.

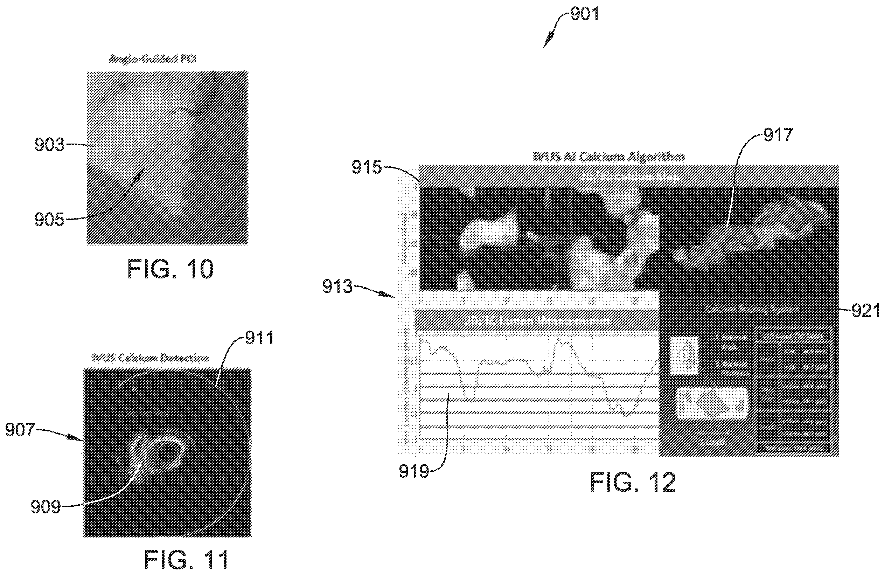

FIGS. 10-12 depict an example display output(s) 901 that may be shown on a display unit. Although each portion/display output is shown and labeled as a separate figure, one or more (or all) of the various display outputs depicted can be on the same or different displays. In other words, all of FIGS. 10-12 can be shown on the same display or the display output(s) shown in FIGS. 10-12 can be display outputs from one or more different displays. The display output 901 may include a fluoroscopy image 903 depicting a portion of a blood vessel 905. On the same display unit or separately, a calcium map 907 may be displayed, illustrating a blood vessel 909 and a representation 911 of a calcification angle/arc. In some instances, an ultrasound line may be included in the representation 911 of a calcification angle/arc (e.g., when the maximal brightness in the lesion segment between the lumen and media borders is much higher than the maximal brightness beyond the media border) to identify ultrasound lines that go through a calcified lesion. In other words, an ultrasound line may be included/added to the representation 911 of a calcification angle/arc to help identify the calcified lesion. On the same display unit or separately, calcium scoring 913 may be shown. Here, a two-dimensional calcium map 915 and/or a three-dimensional calcium map 917 may be shown. A representation 919 of the lumen length may also be shown. Finally, a display 921 representing the calculation of a calcification/calcium score may be shown.

In some instances, the display output 901 may be used to determine a treatment strategy for treating an intravascular lesion in a blood vessel. For example, a first numerical calcium score may be determined by observing the visual representation 911 of a calcium/calcification arc of the intravascular lesion, a two-dimensional calcium map of the blood vessel 915, the three-dimensional calcium map of the blood vessel 917, or combinations thereof. In some instances, a calcification angle/arc that is greater than 180 degrees may get a score of two or three points and a calcification angle/arc that is less than or equal to 180 degrees may get a score of zero or one point. A second numerical calcium score by observing the fluoroscopic image 903 of the blood vessel may be obtained. For example, the fluoroscopic image 903 may be used to determine if the calcified lesion has a thickness of 0.5mm or less. If the calcified lesion has a thickness greater than 0.5mm (e.g., as observed on the fluoroscopic image 903), the second numerical calcium score may be one or two points. If the calcified lesion has a thickness less than or equal to 0.5mm (e.g., as observed on the fluoroscopic image 903), the second numerical calcium score may be zero points. Further, a third numerical calcium score may be determined by measuring a length of the intravascular lesion (e.g., using the representation 919). If the calcified lesion has a length greater than 5mm, the third numerical calcium score may be one or two points. If the calcified lesion has a length less than or equal to 5mm, the third numerical calcium score may be zero or one points. These are just examples. Other calcium scoring parameters are contemplated. A sum of the first numerical calcium score, the second numerical calcium score, and a third calcium score may be determined. A treatment modality may be selected based on the sum. In some instances, the treatment modality may include treatment (e.g., angioplasty) using a non-compliant balloon, treatment (e.g., angioplasty) using a high pressure balloon, treatment (e.g., angioplasty) using a scoring balloon, treatment (e.g., angioplasty) using a cutting balloon, treatment with a rotational atherectomy device, treatment with an orbital atherectomy device, treatment with a laser atherectomy device, treatment with intravascular lithoplasty, combinations thereof, and/or the like.

The processes and/or display output may be used to extract clinically relevant IVUS features, guide treatment strategies such as calcium management, present intuitive maps, and/or combine information on a single display unit or set of display units.

Some example IVUS imaging systems that may be used, for example with the methods disclosed herein, include, but are not limited to, those disclosed in, for example, U.S. Patents Nos. 7,246,959; 7,306,561; and 6,945,938; as well as U.S. Patent Application Publication Nos. US 2006/0100522; US 2006/0106320; US 2006/0173350; US 2006/0253028; US 2007/0016054; and US 2007/003811.

U.S. Patent Application Publication No. US 2015/0073279.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A method for processing intravascular ultrasound images, the method comprising:
receiving, by a processor, collected one or more ultrasound images (517) of a blood vessel;
segmenting the ultrasound images with the processor;
displaying a first image (603a, 603b) of the one or more ultrasound images on a display device; and
displaying a visual representation of a calcification angle on the display device;
wherein the processor includes a deep neural network;
wherein segmenting the ultrasound images includes segmenting with the deep neural network; and
wherein segmenting includes identifying one or more of a lumen border (521) of the blood vessel and a media border (523) for media within the blood vessel, wherein segmenting includes identifying the media border.

2. The method of claim 1, wherein segmenting includes identifying the lumen border.

3. The method of any one of claims 1-2, wherein the deep neural network is trained to identify the lumen border.

4. The method of any one of claims 1-3, wherein the deep neural network is trained to identify the media border.

5. The method of any one of claims 1-4, wherein the deep neural network is trained to identify the lumen border and is trained to identify the media border.

6. The method of claim 1, further comprising displaying a numerical
indication (605a, 605b) of stenosis area for the first image on the display device.

7. The method of claim 6, wherein the numerical indication of stenosis area is displayed on or adjacent to the first image.

8. The method of any one of claims 1-7, further comprising displaying a numerical indication (607a, 607b) of plaque burden for the first image on the display device.

9. The method of any one of claims 1-8, wherein the visual representation of the calcification angle includes an arc disposed along a boundary region of the first image.

10. The method of any one of claims 6-9, further comprising displaying a visual representation of a calcification arc on the display device.

11. The method of claim 10, wherein the visual representation of the calcification arc includes an arc disposed along a boundary region of the first image.

12. The method of claim of any one of claims 1-11,
wherein segmenting includes identifying a cross-sectional area of the blood vessel, a cross-sectional area of the media, the calcification angle of a calcified lesion within the blood vessel, a side branch location, or combinations thereof.

## Patentansprüche

1. Verfahren zur Verarbeitung intravaskulärer Ultraschallbilder, wobei das Verfahren aufweist:
durch einen Prozessor erfolgendes Empfangen eines oder mehrerer erfasster Ultraschallbilder (517) eines Blutgefäßes;
Segmentieren der Ultraschallbilder mit dem Prozessor;
Anzeigen eines ersten Bilds (603a, 603b) des einen oder der mehreren Ultraschallbilder auf einer Anzeigevorrichtung; und
Anzeigen einer visuellen Darstellung eines Verkalkungswinkels auf der Anzeigevorrichtung;
wobei der Prozessor ein tiefes neuronales Netzwerk aufweist;
wobei das Segmentieren der Ultraschallbilder das Segmentieren mit dem tiefen neuronalen Netzwerk aufweist; und
wobei das Segmentieren das Identifizieren einer oder mehrerer Lumengrenzen (521) des Blutgefäßes und einer Mediengrenze (523) für Medien im Blutgefäß aufweist,
wobei das Segmentieren das Identifizieren der Mediengrenze aufweist.

2. Verfahren nach Anspruch 1, wobei das Segmentieren das Identifizieren der Lumengrenze aufweist.

3. Verfahren nach einem der Ansprüche 1 bis 2, wobei das tiefe neuronale Netzwerk trainiert ist, um die Lumengrenze zu identifizieren.

4. Verfahren nach einem der Ansprüche 1 bis 3, wobei das tiefe neuronale Netzwerk trainiert ist, um die Mediengrenze zu identifizieren.

5. Verfahren nach einem der Ansprüche 1 bis 4, wobei das tiefe neuronale Netzwerk trainiert ist, um die Lumengrenze zu identifizieren, und trainiert ist, um die Mediengrenze identifiziert.

6. Verfahren nach Anspruch 1, das ferner das Anzeigen einer numerischen Angabe (605a, 605b) eines Stenoseausmaßes für das erste Bild auf der Anzeigevorrichtung aufweist.

7. Verfahren nach Anspruch 6, wobei die numerische Angabe des Stenoseaumaßes auf dem ersten Bild oder benachbart dazu angezeigt wird.

8. Verfahren nach einem der Ansprüche 1 bis 7, das ferner das Anzeigen einer numerischen Angabe (607a, 607b) einer Plaquebelastung für das erste Bild auf der Anzeigevorrichtung aufweist.

9. Verfahren nach einem der Ansprüche 1 bis 8, wobei die visuelle Darstellung des Verkalkungswinkels einen Bogen aufweist, der entlang einer Grenzregion des ersten Bilds angeordnet ist.

10. Verfahren nach einem der Ansprüche 6 bis 9, das ferner das Anzeigen einer visuellen Darstellung eines Verkalkungsbogens auf der Anzeigevorrichtung aufweist.

11. Verfahren nach Anspruch 10, wobei die visuelle Darstellung des Verkalkungsbogens einen Bogen aufweist, der entlang einer Grenzregion des ersten Bilds angeordnet ist.

12. Verfahren nach einem der Ansprüche 1 bis 11, wobei das Segmentieren das Identifizieren einer Querschnittfläche des Blutgefäßes, einer Querschnittfläche der Medien, des Verkalkungswinkels einer verkalkten Läsion im Blutgefäß, einer Seitenabzweiglage oder Kombinationen daraus aufweist.

## Revendications

1. Procédé de traitement d'images ultrasonores intravasculaires, le procédé comprenant :
la réception, par un processeur, d'une ou plusieurs images ultrasonores (517) collectées d'un vaisseau sanguin ;
la segmentation des images ultrasonores avec le processeur ;
l'affichage d'une première image (603a, 603b) des une ou plusieurs images ultrasonores sur un dispositif d'affichage ; et
l'affichage d'une représentation visuelle d'un angle de calcification sur le dispositif d'affichage ;
dans lequel le processeur inclut un réseau neuronal profond ;
dans lequel la segmentation des images ultrasonores inclut la segmentation avec le réseau neuronal profond ; et
dans lequel la segmentation inclut l'identification d'un ou plusieurs parmi un bord de lumière (521) du vaisseau sanguin et un bord de milieu (523) pour le milieu à l'intérieur du vaisseau sanguin, dans lequel la segmentation inclut l'identification du bord de milieu.

2. Procédé selon la revendication 1, dans lequel la segmentation inclut l'identification du bord de lumière.

3. Procédé selon l'une quelconque des revendications 1 et 2, dans lequel le réseau neuronal profond est entraîné pour identifier le bord de lumière.

4. Procédé selon l'une quelconque des revendications 1 à 3, dans lequel le réseau neuronal profond est entraîné pour identifier le bord de milieu.

5. Procédé selon l'une quelconque des revendications 1 à 4, dans lequel le réseau neuronal profond est entraîné pour identifier le bord de lumière et est entraîné pour identifier le bord de milieu.

6. Procédé selon la revendication 1, comprenant en outre l'affichage d'une indication numérique (605a, 605b) de dimension de sténose pour la première image sur le dispositif d'affichage.

7. Procédé selon la revendication 6, dans lequel l'indication numérique de dimension de sténose est affichée sur ou de façon adjacente à la première image.

8. Procédé selon l'une quelconque des revendications 1 à 7, comprenant en outre l'affichage d'une indication numérique (607a, 607b) de charge de plaque pour la première image sur le dispositif d'affichage.

9. Procédé selon l'une quelconque des revendications 1 à 8, dans lequel la représentation visuelle de l'angle de calcification inclut un arc disposé le long d'une région frontière de la première image.

10. Procédé selon l'une quelconque des revendications 6 à 9, comprenant en outre l'affichage d'une représentation visuelle d'un arc de calcification sur le dispositif d'affichage.

11. Procédé selon la revendication 10, dans lequel la représentation visuelle de l'arc de calcification inclut un arc disposé le long d'une région frontière de la première image.

12. Procédé selon l'une quelconque des revendications 1 à 11,
dans lequel la segmentation inclut l'identification d'une section transversale du vaisseau sanguin, d'une section transversale du milieu, de l'angle de calcification d'une lésion calcifiée à l'intérieur du vaisseau sanguin, d'un emplacement de ramification latérale ou de combinaisons de ceux-ci.
